(19) Europäisches Patentamt
European Patent Office
Office européen des brevets

(11) EP 2 666 800 A1

(12) **EUROPEAN PATENT APPLICATION**

(43) Date of publication:
**27.11.2013 Bulletin 2013/48**

(21) Application number: **13173342.0**

(22) Date of filing: **21.09.2007**

(51) Int Cl.:
***C08G 18/08*** *(2006.01)* ***C08L 75/04*** *(2006.01)*
***C08L 51/00*** *(2006.01)* ***A61K 8/81*** *(2006.01)*
***A61K 8/87*** *(2006.01)* ***C08F 265/04*** *(2006.01)*
***C08G 18/40*** *(2006.01)* ***C08G 18/46*** *(2006.01)*
***C08G 18/75*** *(2006.01)* ***A61Q 5/06*** *(2006.01)*

(84) Designated Contracting States:
**DE ES FR GB IT**

(30) Priority: **22.09.2006 US 846453 P**

(62) Document number(s) of the earlier application(s) in accordance with Art. 76 EPC:
**07253762.4 / 1 903 065**

(71) Applicant: **Rohm and Haas Company**
**Philadelphia,**
**Pennsylvania 19106-2399 (US)**

(72) Inventors:
- **Baxter, Steven Michael**
**Chalfont, PA Pennsylvania 18914 (US)**
- **Schwartz, Curtis**
**Ambler, PA Pennsylvania 19002 (US)**
- **Zeng, Fanwen**
**Belle Mead, NJ New Jersey 08502 (US)**

(74) Representative: **Kent, Venetia Katherine**
**Patent Outsourcing Limited**
**1 King Street**
**Bakewell**
**Derbyshire DE45 1DZ (GB)**

Remarks:
This application was filed on 24-06-2013 as a divisional application to the application mentioned under INID code 62.

(54) **Polymer compositions containing polyurethanes**

(57) There is provided a polymer composition comprising at least one acrylic polymer and at least one sulfonated polyurethane, wherein said acrylic polymer comprises 5% or more by weight, based on the dry weight of said acrylic polymer, polymerized units of at least one carboxyl functional monomer. Also provided are a method of making such a polymer composition and a method of treating hair using such a polymer composition.

EP 2 666 800 A1

## Description

### BACKGROUND:

It is often desired to provide polymer compositions that contain plural polymers of compositions different from each other. For example, it is often useful to provide a polymer composition that contains at least one polyurethane and at least one acrylic polymer. Polyurethanes have unique properties that are different from other polymers such as, for example, polyesters. Sulfonated polyurethanes additionally have desirable stability in water and in other aqueous liquid media, or have desirable miscibility with water, or have both. In addition to physical and chemical properties, sulfonated polyurethanes are relatively easy and inexpensive to manufacture, compared to other sulfonated polymers, such as, for example, sulfonated polyester polymers. Acrylic polymers also provide unique properties. Acrylic polymers that contain carboxyl functional monomer units additionally have further desirable properties, such as, for example, desirable stability in water and in other aqueous liquid media, or desirable miscibility with water, or both.

US Patent 6,093,384 describes hair-care compositions comprising aqueous polymer dispersions, obtained by free-radical polymerization of a radical monomer in the interior and/or partially at the surface of already existing polymer particles of the polyester type.

It is desired to provide a composition that contains sulfonated polyurethane and that contains acrylic polymer that includes carboxyl functional monomer units.

### STATEMENT OF THE INVENTION:

In one aspect of the present invention, there is provided a polymer composition comprising at least one acrylic polymer and at least one sulfonated polyurethane, wherein said acrylic polymer comprises 5% or more by weight, based on the dry weight of said acrylic polymer, polymerized units of at least one carboxyl functional monomer.

### DETAILED DESCRIPTION:

A "polymer," as used herein and as defined by FW Billmeyer, JR. in Textbook of Polymer Science, second edition, 1971, is a relatively large molecule made up of the reaction products of smaller chemical repeat units. Polymers may have structures that are linear, branched, star shaped, looped, hyperbranched, crosslinked, or a combination thereof; polymers may have a single type of repeat unit ("homopolymers") or they may have more than one type of repeat unit ("copolymers"). Copolymers may have the various types of repeat units arranged randomly, in sequence, in blocks, in other arrangements, or in any mixture or combination thereof.

"Polymerizing" herein means the reacting of monomers to form polymer.

Polymerizing may be performed by any type of polymerization process, including, for example, free radical-initiated polymerization and condensation polymerization. Among free radical-initiated polymerization processes, the polymerization method may be, for example, emulsion polymerization, microemulsion polymerization, solution polymerization, bulk polymerization, suspension polymerization, or combinations thereof. In some cases, aqueous emulsion polymerization is performed, and the product is an aqueous polymer latex. Among condensation polymerization processes, the polymerization method may be, for example, solution polymerization, bulk polymerization, continuous phase polymerization, or combinations thereof.

Polymerization methods may also be independently characterized by the method in which monomer is added to the polymerization reaction vessel (i.e., the container in which polymerization takes place). Such addition method may be, for example, batch, semi-batch, shot, gradual addition, or any combination thereof.

Polymer molecular weights can be measured by standard methods such as, for example, size exclusion chromatography (SEC, also called gel permeation chromatography or GPC) or intrinsic viscosity. Generally, polymers have weight-average molecular weight (Mw) of 1,000 or more. Polymers may have extremely high Mw; some polymers have Mw above 1,000,000; typical polymers have Mw of 1,000,000 or less. Some polymers are crosslinked, and crosslinked polymers are considered to have infinite Mw. Some polymers are characterized by Mn, the number-average molecular weight.

Molecules that can react with each other to form the repeat units of a polymer are known herein as "monomers."

One example of a class of monomers that are useful in the present invention are, for example, ethylenically unsaturated monomers (i.e., monomers that have at least one carbon-carbon double bond). Typical ethylenically unsaturated monomers have molecular weight of less than 500. Among such monomers are, for example, vinyl monomers, which are molecules that have at least one vinyl group (i.e.,

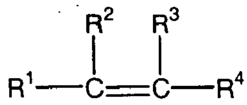
,

where each of $R^1$, $R^2$, $R^3$, and $R^4$ is, independently, a hydrogen, a halogen, an aliphatic group (such as, for example, an alkyl group), a substituted aliphatic group, an aryl group, a substituted aryl group, another substituted or unsubstituted organic group, or any combination thereof). Some suitable vinyl monomers include, for example, styrene, substituted styrenes, dienes, ethylene, ethylene derivatives, and mixtures thereof. Ethylene derivatives include, for example, unsubstituted or substituted versions of the following: vinyl acetate, acrylonitrile, (meth)acrylic acids, (meth)acrylates, (meth) acrylamides, vinyl chloride, halogenated alkenes, and mixtures thereof. As used herein, "(meth)acrylic" means acrylic or methacrylic; "(meth)acrylate" means acrylate or methacrylate; and "(meth)acrylamide" means acrylamide or methacrylamide. "Substituted" means having at least one attached chemical group such as, for example, alkyl group, alkenyl group, vinyl group, hydroxyl group, carboxylic acid group, other functional groups, and combinations thereof. In some embodiments, substituted monomers include, for example, monomers with more than one carbon-carbon double bond, monomers with hydroxyl groups, monomers with other functional groups, and monomers with combinations of functional groups.

A polymer that is made by polymerizing a certain monomer, either alone or with other monomers, is said herein to include that monomer as a monomer unit.

In some embodiments, the present invention involves the use of one or more chain transfer agent. Chain transfer agents are compounds capable of participating in a chain transfer reaction during radical polymerization of monomer.

As used herein, "acrylic monomer" is any monomer selected from (meth)acrylic acid, esters of (meth)acrylic acid, amides of (meth)acrylic acid, substituted versions thereof, and mixtures thereof.

Acrylic polymers are polymers that have monomer units that are 50% or more acrylic monomers by weight, based on the weight of the polymer. Some acrylic polymers have 75% or more, or 80% or more, or 90% or more acrylic monomer units by weight, based on the weight of the polymer. In some cases, acrylic polymers include copolymerized monomer units of monomers that are vinyl monomers other than acrylic monomers. Vinyl monomers other than acrylic include, for example, styrene, substituted styrenes, vinyl esters of organic acids, N-vinyl compounds, dienes, maleic acid, maleic anhydride, other unsaturated dicarboxylic acids or their anhydrides, and mixtures thereof.

As used herein, a "polyurethane" is a substance containing two or more urethane linking groups per molecule in the main chain. A urethane linking group has the structure

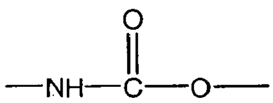
.

Normally, a polyurethane is the reaction product of at least one polyol with at least one polyisocyanate. A polyol is a substance with two or more hydroxyl groups per molecule. A polyisocyanate is a substance with two or more isocyanate groups per molecule. Some polyurethanes are reaction products of at least one polyol, at least one polyisocyanate, and at least one additional reactant such as, for example a polyamine, an aminoalcohol, or a mixture thereof. A polyamine is a substance with two or more amine groups per molecule. When an isocyanate group reacts with an amine group, a urea link is formed. Some polyurethanes are made without the use of polyamines. An aminoalcohol is a substance with at least one amino group and at least one hydroxyl group. Some polyurethanes are made without the use of aminoalcohols. Some polyurethanes are made without polyamines and without aminoalcohols. Some polyurethanes are made without any reactants other than polyisocyanates and polyols.

As used herein, a "hybrid polymer composition" is a composition resulting from polymerizing a second polymer in the presence of a first polymer, where the composition contains both the first polymer and the second polymer, which may or may not be fully or partially covalently bound to each other. Hybrid polymer compositions include, for example, compositions in which the first polymer is a polyurethane and the second polymer is an acrylic polymer. Hybrid polymer compositions include, for another example, compositions in which the first polymer is an acrylic polymer and the second polymer is a polyurethane. Some hybrid polymer compositions have three or more polymers.

In some embodiments, the composition of the present invention further includes a continuous liquid medium. In some of such embodiments, acrylic polymer and sulfonated polyurethane are carried in the continuous liquid medium. A material is "carried" in a continuous liquid medium if that material is either dissolved in the continuous liquid medium or dispersed in the continuous liquid medium or a combination thereof.

As used herein, a composition is "aqueous" if it contains 25% or more water by weight based on the weight of the composition. Some aqueous compositions contain 40% or more; or 50% or more; water by weight, based on the

weight of the composition. In some aqueous compositions, water forms a continuous medium, and one or more other substance is dissolved or dispersed in the continuous liquid medium. In aqueous compositions in which water forms a continuous liquid medium, the water may or may not be mixed with one or more additional liquids other than water that are miscible with water. In some aqueous compositions with a continuous liquid medium, the continuous liquid medium contains 25% or more water; or 50% or more water; or 75% or more water; or 90% or more; water, by weight based on the weight of the continuous liquid medium. A continuous liquid medium containing 25% or more water, by weight based on the weight of the continuous medium, is known herein as an aqueous medium.

A material is said herein to be "dispersed" in a continuous liquid medium when that material exists as discrete particles distributed through the continuous liquid medium. The discrete particles may be solid, liquid, gas, or a combination thereof. The dispersion may be, for example, in the form of an emulsion, miniemulsion, microemulsion, suspension, latex, or combination thereof.

As used herein, "Tg" is the glass transition temperature of a polymer, as measured by Differential Scanning Calorimetry. Tg measurements are performed on polymer samples that do not have absorbed non-polymeric material (such as, for example, water or plasticizer). Some polymers have exactly one Tg. Some polymers have two or more Tg's.

Herein, some compounds that are used in the practice of the present invention are described as reaction products of certain ingredients. Unless otherwise stated in specific cases, it is to be understood that such a compound is contemplated for use in the present invention if it is actually made by reacting the certain ingredients; an identical compound, even if it is made by a different method, is also contemplated.

Herein, when a ratio is said to be "X:1 or higher," it is meant that the ratio is Y:1, where Y is larger than or equal to X. Similarly, when a ratio is said to be "P:1 or lower," it is meant that the ratio is Q:1, where Q is smaller than or equal to P.

As used herein, a sulfonated compound is a compound with one or more sulfonate groups per molecule. A sulfonate group is $-SO_3^{\ominus}$. Depending on the environment surrounding a sulfonate group, it may exist as an anion, which may be solvated or coordinated with a cation or both, or it may exist as a sulfonic acid group, i.e., $-SO_3H$. The sulfonate group may be introduced by any means.

The practice of the present invention involves the use of one or more sulfonated polyurethane. Sulfonated polyurethane may be made by any method. For example, at least one sulfonated polyol may be reacted with at least one polyisocyanate and, optionally, at least one additional polyol. For another example, at least one amino-sulfonic acid compound may be reacted with at least one isocyanate-terminal polyurethane; such an isocyanate-terminal polyurethane may have no sulfonate groups or may have one or more sulfonate groups.

When a sulfonated polyol is used for making the sulfonated polyurethane, one useful class of sulfonated polyols is, for example, sulfonated polyester polyols. Sulfonated polyester polyols may be made by any method. Some suitable sulfonated polyester polyols are those, for example, described in US Patents 5,698,626 and 5,753,774.

Polyisocyanates which may be used in making sulfonated polyurethanes include, for example, aromatic polyisocyanates, aliphatic polyisocyanates, cycloaliphatic polyisocyanates, and combinations thereof. Some embodiments use one or more of the following polyisocyanates: 4,4'-diphenylmethane diisocyanate (also called 4, 4'-methylene bisphenyl diisocyanate or 4,4'-MDI); 2,4'-diphenylmethane diisocyanate (also called 2, 4'-methylene bisphenyl diisocyanate or 2,4'-MDI); any isomer of diisocyanato toluene (TDI); polymeric MDI; naphthalene-1,5-diisocyanate (also called NDI); 1,6-hexamethylene diisocyanate (also called HDI); 1-isocyanato-3-isocyanatomethyl-3,5,5-trimethyl-cyclohexane (also called isophorone diisocyanate or IPDI); and mixtures thereof. Some embodiments use "pure MDI," which is a mixture of 4,4'-MDI with 2,4'-MDI that has a ratio of 4,4'-MDI to 2,4'-MDI of approximately 98/2 by weight. Some embodiments use IPDI. Some embodiments use IPDI as the sole polyisocyanate.

Some suitable sulfonated polyurethanes are, for example, reaction products of at least one isocyanate-terminal polyurethane with at least one amino-sulfonic acid compound. An isocyanate-terminal polyurethane is a polyurethane in which at least 50% of the polyurethane molecules, on a molar basis, have two or more isocyanate groups. Isocyanate-terminal polyurethanes are sometimes referred to as "prepolymers." One typical way to make an isocyanate-terminal polyurethane is to react a diol with a diisocyanate, using a molar excess of diisocyanate. An amino-sulfonic acid compound is a compound that has an amine group (i.e., $-NH_2$) and a sulfonic acid group. One example of an amino-sulfonic acid compound is 2-((2-aminoethyl)amino) ethanesulfonic acid. Further examples of amino-sulfonic acid compounds are adducts of ethylenically unsaturated sulfonic acids compounds with compounds having an amine group and a second group that has an active hydrogen atom. One example of an ethylenically unsaturated sulfonic acid compound is 2-acrylamido-2-methylpropanesulfonic acid. A group with an active hydrogen atom is a group capable of acting as a "donor" in a carbon Michael addition reaction. Examples of groups with active hydrogen atoms are amine groups and hydroxyl groups. Compounds having an amine group and a second group that has an active hydrogen atom include, for example, diamines (i.e., compounds with two amine groups) and aminoalcohols (i.e., compounds with an amine group and a hydroxyl group). It is contemplated that amino-sulfonic compounds can be formed, for example, by performing a carbon Michael reaction between the second group that has an active hydrogen atom and the ethylenic unsaturation of the

unsaturated sulfonic acid compound.

Some methods of making sulfonated polyurethanes using amino-sulfonic acid compounds are described in US Patent 5,629,402. Independently, some sulfonated polyurethanes are described, for example, in US 6,517,821.

A sulfonated polyurethane may be made using any method. In some embodiments, a sulfonated polyurethane is made in solution in an organic solvent. Independently, in some embodiments, a sulfonated polyurethane is used in the form of an aqueous dispersion or in the form of a solution in an aqueous medium. Among embodiments in which the sulfonated polyurethane is in the form of an aqueous dispersion, the sulfonated polyurethane may be dispersed in an aqueous medium by any method. In some of such embodiments, for example, a sulfonated polyurethane is made by solution polymerization in an organic solvent, then the organic solvent is removed, and then the sulfonated polyurethane is dispersed in an aqueous medium.

In some embodiments, a sulfonated polyurethane is present in a composition that has an aqueous medium. The sulfonated polyurethane may be dissolved in the aqueous medium or may dispersed in the aqueous medium in the form of discrete particles, or may be present as a mixture of dissolved molecules and dispersed particles.

In some embodiments, sulfonated polyurethane is present as particles dispersed in aqueous medium. The particle size of such a dispersion can be measured, for example by Capillary Hydrodynamic Fractionation (CHDF). In some embodiments, the median particle size is 10 nm or greater, or 20 nm or greater. Independently, in some embodiments, the median particle size is 200 nm or smaller, or 100 nm or smaller, or 50 nm or smaller. In dependently, in some embodiments, the distribution of particle sizes is unimodal; that is, in the distribution of amount of material versus particle size, there is one clear principal peak, and any other peak (if any) that exists has height less that 5% of the height of the principal peak.

Independent of the physical form of a sulfonated polyurethane, that sulfonated polyurethane can be characterized by molecular weight analysis, for example using GPC. It is contemplated that using GPC normally involves identifying and using, as an eluent, a solvent that dissolves the sulfonated polyurethane. In some embodiments, sulfonated polyurethane has weight-average molecular weight of 5,000 or greater, or 7,500 or greater. Independently, in some of such embodiments, sulfonated polyurethane has weight-average molecular weight of 200,000 or smaller, or 100,000 or smaller, or 50,000 or smaller.

Another independent aspect of a sulfonated polyurethane is the amount of sulfonate groups. This amount is also called the "acid amount." This amount is reported in units of milliequivalents of sulfonate groups per 100 g of polyurethane solids. In some embodiments, sulfonated polyurethane has sulfonate groups in the amount, in milliequivalents per 100 g of polyurethane solids, of 10 or more; or 20 or more; or 30 or more; or 40 or more. Independently, in some embodiments, sulfonated polyurethane has sulfonate groups in the amount, in milliequivalents per 100 g of polyurethane solids, of 150 or less; or 100 or less; or 80 or less.

The practice of the present invention involves use of at least one acrylic polymer. In some embodiments, an acrylic polymer is present with a Tg of 15°C or higher; or 25°C or higher; or 35°C or higher; or 45°C or higher. Independently, in some embodiments, and acrylic polymer is present with a Tg of 110°C or lower; or 95°C or lower; or 80°C or lower; or 70°C or lower.

Any acrylic monomer or mixture thereof is suitable as monomer units included in acrylic polymer. In some embodiments, the monomers include one or more alkyl (meth)acrylate (i.e., an alkyl ester of acrylic acid or methacrylic acid). Alkyl group may be linear or branched or cyclic or a combination thereof. In some embodiments, at least one alkyl (meth)acrylate is used in which the alkyl group has one carbon atom, or two carbon atoms, or three carbon atoms, or four carbon atoms, or five or more carbon atoms. Independently, at least one alkyl (meth)acrylate is used in which the alkyl group has 20 or fewer carbon atoms, or 10 or fewer carbon atoms, or 8 or fewer carbon atoms.

In some embodiments, at least one alkyl methacrylate monomer is used. One suitable alkyl methacrylate monomer is methyl methacrylate. When an alkyl methacrylate monomer is used, in some embodiments the amount of alkyl methacrylate monomer is, by weight based on the weight of the acrylic polymer, 10% or more, or 20% or more, or 35% or more. Independently, when an alkyl methacrylate monomer is used, in some embodiments the amount of alkyl methacrylate monomer is, by weight based on the weight of the acrylic polymer, 80% or less, 70% or less, or 60% or less. In some embodiments, an acrylic polymer is used that has no monomer units of alkyl methacrylate.

Independently, in some embodiments, at least one alkyl acrylate monomer is used. Some suitable alkyl acrylate monomers have alkyl groups with 2 or more carbon atoms. One suitable alkyl acrylate monomer is n-butyl acrylate. When an alkyl acrylate monomer is used, in some embodiments the amount of alkyl acrylate monomer is, by weight based on the weight of the acrylic polymer, 5% or more, or 10% or more, or 15% or more. Independently, when an alkyl acrylate monomer is used, in some embodiments the amount of alkyl acrylate monomer is, by weight based on the weight of the acrylic polymer, 90% or less, or 80% or less, or 70% or less, or 60% or less, or 50% or less, or 40% or less.

Independently, in some embodiments, at least one substituted alkyl (meth)acrylate monomer is used. A substituted alkyl (meth)acrylate is an alkyl ester of (meth)acrylic acid in which the alkyl group has at least one substituent group. A substituent group is any chemical group containing at least one atom other than carbon and hydrogen. One suitable substituent group is the hydroxyl group. Some suitable substituted alkyl (meth)acrylates are, for example,

hydroxypropyl (meth)acrylate, hydroxyethyl (meth)acrylate, and mixtures thereof. In some embodiments, at least one hydroxyalkyl methacrylate monomer is used. When a substituted alkyl (meth)acrylate monomer is used, in some embodiments the amount of substituted alkyl (meth)acrylate monomer is, by weight based on the weight of the acrylic polymer, 2% or more, or 5% or more, or 8% or more. Independently, when a substituted alkyl (meth)acrylate monomer is used, in some embodiments the amount of substituted alkyl (meth)acrylate monomer is, by weight based on the weight of the acrylic polymer, 50% or less, 30% or less, or 20% or less.

The practice of the present invention involves the use of at least one carboxyl functional monomer. A carboxyl functional monomer is an ethylenically unsaturated monomer that also contains at least one carboxylic acid group or at least one anhydride group. A carboxyl functional monomer may have one or more than one carbon-carbon double bond. Independently, a carboxyl functional monomer may have one carboxylic acid group or more than one carboxylic acid group. The carboxylic acid group or groups may be in the form of a neutral carboxylic acid group, in the form of a corresponding anion, in the form of an anhydride, or any combination or mixture thereof. Some carboxyl functional monomers include, for example, (meth)acrylic acid, substituted (meth)acrylic acid, carboxyl functional alkenes, and mixtures thereof. In some embodiments, carboxyl functional monomers include one or more of acrylic acid, methacrylic acid, crotonic acid, itaconic acid, fumaric acid, maleic acid, monomethyl itaconate, monomethyl maleate, monobutyl maleate, maleic anhydride, and mixtures thereof. In some embodiments carboxyl functional monomer includes acrylic acid, methacrylic acid, or mixtures thereof. The amount of carboxyl functional monomer is, by weight based on the weight of the acrylic polymer, 5% or more, or 7% or more, or 10% or more. Independently, the amount of carboxyl functional monomer is, by weight based on the weight of the acrylic polymer, 30% or less, or 25% or less, or 20% or less.

In some embodiments, one or more chain transfer agent is used. Some suitable chain transfer agents are, for example, halomethanes, disulfides, thiols (also called mercaptans), and metal complexes. Also suitable as chain transfer agents are various other compounds that have at least one readily abstractable hydrogen atom. Mixtures of suitable chain transfer agents are also suitable. In some embodiments, at least one mercaptan is used. In some embodiments, n-dodecyl mercaptan is used. When a chain transfer agent is used, in some embodiments the amount of chain transfer agent is, by weight based on the weight of the acrylic polymer, 0.02% or more, or 0.05% or more, or 0.1% or more, or 0.3% or more, or 0.8% or more. Independently, when a chain transfer agent is used, in some embodiments the amount of chain transfer agent is, by weight based on the weight of the acrylic polymer, 5% or less, 2.5% or less, or 1.5% or less.

In some embodiments, the acrylic polymer contains few or no monomer units of amide monomers. Amide monomers include, for example, N-vinyl pyrrolidone, (meth)acrylamide, N-alkyl (meth)acrylamide, other amide monomers, and mixtures thereof. In some embodiments, the acrylic polymer contains 1% or less, by weight based on the weight of the acrylic polymer, amide monomer units. In some embodiments, the acrylic polymer contains no amide monomer units.

The acrylic polymer may be formed by any method. Some suitable methods include, for example, bulk polymerization, solution polymerization, emulsion polymerization, suspension polymerization, and combinations thereof. One type of polymerization that is used for forming an acrylic polymer is, for example, free radical polymerization. One method of conducting free radical polymerization is, for example, aqueous emulsion polymerization. Any method of aqueous emulsion polymerization may be used. Aqueous emulsion polymerization involves monomer, water-soluble initiator, and surfactant-functional compound in the presence of water.

In some embodiments, at least one surfactant-functional compound is used during aqueous emulsion polymerization that is selected from common surfactants such as, for example, anionic surfactants, cationic surfactants, and nonionic surfactants. Some suitable common surfactants are, for example, alkyl sulfates, alkylaryl sulfates, alkyl or aryl polyoxyethylene nonionic surfactants, and mixtures thereof.

Aqueous emulsion polymerization may be performed with any water soluble initiator. Suitable initiators include, for example, water soluble peroxides, such as, for example, sodium or ammonium persulfate. Suitable initiators also include, for example, oxidants (such as, for example, persulfates or hydrogen peroxide) in the presence of reducing agents (such as, for example, sodium bisulfite or isoascorbic acid) and/or polyvalent metal ions, to form an oxidation/reduction pair to generate free radicals at any of a wide variety of temperatures.

In some embodiments, aqueous emulsion polymerization is performed by a method that includes forming at least one monomer emulsion in one vessel and then adding that monomer emulsion to a separate polymerization reaction vessel in which polymerization takes place. In some of such embodiments, monomer emulsion is formed by mixing one or more monomer, surfactant-functional compound, and water to form dispersed droplets of monomer. In such embodiments, initiator may be added to the polymerization reaction vessel before, during, or after monomer emulsion is added, or any combination thereof.

In some embodiments, neat monomer is added to the polymerization reaction vessel. Neat monomer is monomer or mixture of monomers to which no other ingredient (such as, for example, water or surfactant) has been added. Independently, if more than one monomer is used, monomers may be added to the polymerization reaction vessel as one or more mixtures or as separate addition streams or as a combination thereof. In some of such embodiments, some or all of the at least one water-soluble initiator that will be used in the polymerization process is placed into a polymerization

reaction vessel, and then polymerization occurs when neat monomer is added to the polymerization reaction vessel. In some of such embodiments, neat monomer is added gradually to the polymerization reaction vessel while polymerization takes place in the polymerization reaction vessel.

In some embodiments of the practice of the present invention, the acrylic polymer that results from aqueous emulsion polymerization is in the form of a latex.

Suitable acrylic polymer may be a single stage polymer or a multistage polymer. As used herein, a "multistage" polymer is a polymer that is made in more than one polymerization stage. A polymerization stage is a process in which polymerization takes place and then effectively ends. That is, at the end of a polymerization stage, little or no monomer is present (i.e., the amount of remaining monomer is 10% or less, or 5% or less, or 2% or less, by weight based on the weight of polymer produced by that polymerization stage), and the rate of polymerization is negligible or zero. In a multistage polymerization process, after the first stage is ended, a second stage is conducted in the presence of the polymer made by the previous stage. Optionally, one or more additional polymerization stage may be conducted after the second stage; each stage is performed after the previous polymerization stage has effectively ended.

In some embodiments involving a multistage acrylic polymer, the composition of the acrylic polymer made during the second stage is different from the composition of the acrylic polymer made during the first stage. In some embodiments, some or all of the acrylic polymer made in the first stage is left in place in the vessel in which the first stage was conducted, and the second stage is conducted in the same vessel. In some embodiments, the acrylic polymer made in the first stage is removed and placed in a new container, with or without dilution by water, and the second stage is performed in the new container. After the second stage, further stages may or may not be conducted.

In some embodiments, the first stage is an emulsion polymerization process that produces an acrylic polymer latex. In some of such embodiments, when a second stage is conducted, most or all of the acrylic polymer produced in the second stage is formed on, in, or attached to the acrylic latex particles made in the first stage. Thus, the result is a latex in which most or all of the particles each contain acrylic polymer from the first stage and acrylic polymer from the second stage. If subsequent stages are conducted, in some embodiments, some or all of the polymer from each subsequent stage will form on, in, or attached to the particles formed in the previous stage.

In some embodiments that involve a multistage acrylic polymer, at least one of the stages produces an acrylic polymer that is a soft polymer. A soft polymer is a polymer with a Tg of 40°C or lower. In some embodiments, a soft polymer is used that has a Tg of -50°C or higher; or -25°C or higher; or 0°C or higher; or 25°C or higher. In some embodiments, at least one soft polymer is used that has only one glass transition temperature.

In some embodiments that involve a multistage acrylic polymer, at least one of the stages produces an acrylic polymer that is a hard polymer. A hard polymer is a polymer with a Tg of higher than 40°C. In some embodiments, a hard polymer is used that has a Tg of 60°C or higher; or 80°C or higher. Independently, in some embodiments, a hard polymer is used that has a Tg of 200°C or lower; or 150°C or lower; or 120°C or lower. In some embodiments, at least one hard polymer is used that has only one glass transition temperature.

In some embodiments that involve a multistage acrylic polymer, at least one of the stages produces an acrylic polymer that is a hard polymer, and at least one of the stages produces an acrylic polymer that is a soft polymer. In some of such embodiments, the Tg of the hard polymer is at least 10°C higher than the Tg of the soft polymer. In some embodiments, the Tg of the hard polymer is at least 20°C higher, or at least 30°C higher, or at least 40°C higher, or at least 50°C higher, than the Tg of the soft polymer. Independently, in some of such embodiments, at least one hard polymer and at least one soft polymer are used in amounts such that the weight ratio of hard polymer to soft polymer is from 1.01:1 to 100:1. In some embodiments, the weight ratio of hard polymer to soft polymer is 1.05:1 or higher ; or 1.1:1 or higher; or 1.2:1 or higher; or 1.3:1 or higher; or 1.4:1 or higher. In some embodiments, the weight ratio of hard polymer to soft polymer is 4:1 or lower; or 3:1 or lower; or 2:1 or lower; or 1.6:1 or lower.

The composition of the present invention may be made by any method. In some embodiments, for example, at least one sulfonated polyurethane is mixed with at least one acrylic polymer. In some embodiments, all or a portion of an acrylic polymer is made in the presence of a sulfonated polyurethane. In some embodiments, all or a portion of a sulfonated polyurethane is made at the same time as all or a portion of an acrylic polymer.

In some embodiments, the ratio of the dry weight of sulfonated polyurethane to dry weight of acrylic polymer is 0.1:1 or higher; or 0.2:1 or higher; or 0.4:1 or higher. Independently, in some embodiments, the ratio of the dry weight of sulfonated polyurethane to dry weight of acrylic polymer is 3:1 or lower; or 2:1 or lower; or 1:1 or lower.

In some embodiments, at least one sulfonated polyurethane is made in the absence of acrylic polymer, and at least one acrylic polymer is made in the absence of sulfonated polyurethane, and then the sulfonated polyurethane is mixed with the acrylic polymer. In some of such embodiments, the sulfonated polyurethane is in the form of an aqueous dispersion. Independently, in some of such embodiments, the acrylic polymer is an aqueous latex. Also contemplated are embodiments in which such an aqueous dispersion of a sulfonated polyurethane is mixed with such an aqueous latex acrylic polymer. Additionally contemplated are embodiments in which such a sulfonated polyurethane is made as an aqueous dispersion, such an acrylic polymer is made as an aqueous latex, and then a solution is formed in a mixture of water and a water-miscible liquid other than water, where the solution contains that acrylic polymer and that sulfonated

polyurethane.

In some embodiments, a sulfonated polyurethane is in neat form, in the form of a solution, in the form of a dispersion, or a combination thereof. In some embodiments, independent of the form of sulfonated polyurethane, an acrylic polymer is in neat form, in the form of a solution, in the form of a dispersion, or a combination thereof.

In some embodiments, a sulfonated polyurethane is used in the form of an aqueous dispersion. In some of such embodiments, some or all acrylic polymer is present in such a form that some or all of the particles of the aqueous dispersion of sulfonated polyurethane additionally contain acrylic polymer. Among embodiments that contain aqueous dispersion in which some or all particles contain both sulfonated polyurethane and acrylic polymer, it is contemplated that in some of such embodiments there is also present acrylic polymer that is not in any particle that contains sulfonated polyurethane; such acrylic polymer may be, for example, in solution, in other particles in aqueous dispersion, or a combination thereof. Independently, among embodiments that contain aqueous dispersion in which some particles contain both sulfonated polyurethane and acrylic polymer, it is contemplated that in some of such embodiments there is also present particles that contain sulfonated polyurethane but no acrylic polymer, or sulfonated polyurethane in solution, or a combination thereof.

In some embodiments of the present invention, at least one acrylic polymer is formed in the presence of at least one aqueous dispersion of at least one sulfonated polyurethane. The acrylic polymer may be formed by any method. Regardless of the particular polymerization method used, the composition resulting from forming acrylic polymer in the presence of at least one aqueous dispersion of at least on sulfonated polyurethane is a hybrid polymer composition. In some embodiments, at least one acrylic polymer is formed using aqueous emulsion polymerization in the presence of at least one sulfonated polyurethane that is in the form of an aqueous dispersion. Any method of aqueous emulsion polymerization may be used.

It is contemplated that, in some embodiments, at least one sulfonated polyurethane functions as a surfactant-functional compound during aqueous emulsion polymerization. In some embodiments, aqueous emulsion polymerization of acrylic polymer is performed in the presence of a dispersion of at least one sulfonated polyurethane, and no anionic surfactant other than sulfonated polyurethane is present during polymerization of acrylic polymer. In some embodiments, aqueous emulsion polymerization of acrylic polymer is performed in the presence of a dispersion of at least one sulfonated polyurethane, and no nonionic surfactant is present during polymerization of acrylic polymer. In some embodiments, aqueous emulsion polymerization of acrylic polymer is performed in the presence of a dispersion of at least one sulfonated polyurethane, and no surfactant-functional compound other than sulfonated polyurethane is present during polymerization of acrylic polymer.

In some embodiments, aqueous dispersion of at least one sulfonated polyurethane is placed into a polymerization reaction vessel along with at least one water-soluble initiator, without any surfactant-functional compound other than the one or more sulfonated polyurethane, and then polymerization occurs when monomer is added to the polymerization reaction vessel. In some of such embodiments, monomer is added gradually to the polymerization reaction vessel while polymerization takes place in the polymerization reaction vessel.

In some embodiments, sulfonated polyurethane is present in the form of an aqueous dispersion. In some of such embodiments, carboxyl groups are added to the aqueous dispersion, either as carboxyl functional monomers or as carboxyl groups attached to monomer units of.an acrylic polymer. When such carboxyl groups are added, it is sometimes desirable that the carboxyl groups remain in neutral form (i.e., in protonated form), while the sulfonate groups remain in anionic form. That is, it is sometimes desirable that sulfonated polyurethane is stronger acid than the carboxyl groups.

One useful measure of the strength of an acid is the acid dissociation constant, which is characterized by pKa. Stronger acids have lower values of pKa.

In some embodiments, for example, sulfonated polyurethane has pKa value lower than the pKa values of the carboxyl groups. In some of such embodiments, pH of the aqueous medium is adjusted to be higher than the pKa of the sulfonated polyurethane and lower than the pKa value of the carboxyl groups. Such a procedure is useful, for example, when performing emulsion polymerization of acrylic monomer in the presence of sulfonated polyurethane.

It can be useful to characterize "delta-pKa," the difference calculated by subtracting the pKa value of the sulfonated polyurethane (or, if more than one sulfonated polyurethane is used, the highest pKa value thereof) from the pKa value of the carboxyl functional monomer or of the acrylic polymer that contains monomer units of carboxyl functional monomer (or, if more than one carboxyl functional monomer or acrylic polymer is used, the lowest pKa value thereof). In some embodiments, delta-pKa is one unit or more; or two units or more; or three units or more; or four units or more.

In some embodiments involving acrylic polymer formed by emulsion polymerization in the presence of an aqueous dispersion of at least one sulfonated polyurethane, at the conclusion of emulsion polymerization of acrylic polymer, the composition contains little or no gel.

Gel is solid material that forms during emulsion polymerization of acrylic polymer or forms within 30 minutes of the completion of emulsion polymerization of acrylic polymer. Gel is made of particles much larger than latex particles. Gel particles are 1 micrometer or larger; or 5 micrometer or larger; or 25 micrometer or larger, or 100 micrometer or

larger. Gel particles may float, may remain dispersed, may settle to the bottom of the container, or any combination thereof. Gel can be removed from the composition by filtration, for example by passing the composition through a sequence of screens with decreasing opening sizes (for example, a screen of 20 mesh number, followed by a screen of 100 mesh number, followed by a screen of 325 mesh number). The amount of gel is characterized by grams of gel per liter (or per quart) of total composition. In some embodiments, the amount of gel is 1.06 gram/liter (1 gram/quart) or less; or 0.53 gram/liter (0.5 gram/quart) or less; or 0.2 gram/quart or less. In some embodiments, no gel is detected.

In some embodiments, the composition of the present invention is made by a method that includes polymerization of acrylic polymer in the presence of at least one dispersion of at least one sulfonated polyurethane. In some of such embodiments, the acrylic polymer that results is in the form of a latex. In some embodiments, the latex contains particles that each contains acrylic polymer and sulfonated polyurethane, and such particles are called herein "hybrid latex" particles. In some of such embodiments, some of the acrylic polymer particles may possibly exist without sulfonated polyurethane, and, independently, some of the sulfonated polyurethane may possibly exist somewhere in the composition other than as part of a particle that contains acrylic polymer. In embodiments in which some or all of the acrylic polymer latex particles contain sulfonated polyurethane, the sulfonated polyurethane may exist in the interior of the particle, on the surface of the particle, or a combination thereof. Independently, in embodiments in which some or all of the acrylic polymer latex particles contain sulfonated polyurethane, some or all of the sulfonated polyurethane in the acrylic polymer latex particle may be covalently bound to acrylic polymer, or some or all of the sulfonated polyurethane in the acrylic polymer latex particle may be present in the acrylic polymer latex particle without being covalently bound to acrylic polymer, or some sulfonated polyurethane in the acrylic latex particle may be bound to acrylic polymer while some sulfonated polyurethane in the acrylic latex particle is not covalently bound to acrylic polymer.

Among embodiments involving hybrid latex particles, it is sometimes useful to characterize the percent solids of the latex. Percent solids of a composition is measured by providing an initial portion of the composition, allowing that initial portion to fully evaporate (i.e., to reach equilibrium with air at 25°C and 0% relative humidity), and weighing the material that has not evaporated. The weight of the material that has not evaporated, as a percentage of the total weight of the initial portion of the composition, is the percent solids.

In some embodiments involving hybrid latex particles, the percent solids of the latex is 5% or greater; or 10% or greater; or 20% or greater; or 25% or greater. Independently, in some embodiments, the percent solids of the latex is 60% or less; or 50% or less; or 45% or less.

In some embodiments involving hybrid latex particles, the composition of the aqueous medium is, by weight based on the weight of the aqueous medium, 75% water or more; or 85% water or more; or 90% water or more; or 95% water or more; or 98% water or more.

Among embodiments involving hybrid latex particles, the distribution of particle sizes can be studied by CHDF. In some of such embodiments, the mean particle size is 25 nm or greater, or 40 nm or greater, or 50 nm or greater. Independently, in some of such embodiments, the mean particle size is 300 nm or smaller, or 200 nm or smaller, or 100 nm or smaller. Independently, in some of such embodiments, the particle size distribution of the polymer latex particles is unimodal.

In some embodiments involving hybrid polymer latex particles, the hybrid polymer has weight-average molecular weight of 1,000 or greater, or 2,000 or greater, or 5,000 or greater. Independently, in some of such embodiments, hybrid polymer has weight-average molecular weight of 200,000 or smaller, or 100,000 or smaller, or 75,000 or smaller.

In some embodiments involving a continuous liquid medium, the continuous liquid medium is water or a mixture of water and a water-miscible liquid other than water. Some suitable water-miscible liquids are, for example, alkyl alcohols, where the alkyl group is linear or branched and, independently, where the alkyl group has 1 to 6 carbon atoms. One example of a suitable alcohol is ethanol. In some embodiments, the continuous liquid medium has 80% water or less, by weight based on the weight of the continuous liquid medium; or 75% water or less; or 60% water or less; or 30% water or less; or 19% water or less; or 5% water or less; or 1% water or less. Independently, in some embodiments, the continuous liquid medium has 0.01% water or more, by weight based on the weight of the continuous liquid medium; or 1% water or more; 10% water or more; or 20% water or more; or 40% water or more. Also contemplated are embodiments in which the continuous liquid medium has 99% water or more, by weight based on the weight of the continuous liquid medium. Independently, further contemplated are embodiments in which the continuous liquid medium has 0.1% water or less, by weight based on the weight of the continuous liquid medium. In some embodiments, a continuous liquid medium is used that contains no water.

Among embodiments involving a continuous liquid medium that contains 5% or less water, by weight based on the weight of the continuous liquid medium, the continuous liquid medium contains one or more liquids other than water, and each of these liquids, independent of each other, may or may not be water-miscible.

Among embodiments in which a water-miscible liquid other than water is used, the water-miscible liquid may be, for example, a cosmetically acceptable liquid. "Cosmetically acceptable" means herein a material that is suitable for contact with the human body.

Some embodiments involve a formulation with the composition of the present invention in a continuous liquid

medium that is a mixture of water and a water-miscible liquid other than water, where the formulation has relatively low percent solids. Among such embodiments, the amount of water in the continuous liquid medium may be, for example, by weight based on the weight of the continuous liquid medium, 60% or less; or 50% or less; or 45% or less. Independently, the amount of water in the continuous liquid medium may be, for example, by weight based on the weight of the continuous liquid medium, 25% or more; or 30% or more; or 35% or more. Independently, the percent solids of such a formulation may be, for example, 0.5% or greater; or 1% or more; or 2% or more; or 4% or more. Independently, the percent solids of such a formulation may be, for example, 15% or less; 10% or less; or 8% or less; or 6% or less.

In some embodiments, the composition of the present invention is made and used as follows. In these particular embodiments, an aqueous dispersion of sulfonated polyurethane is provided. Then, an acrylic polymer that includes monomer units of at least one carboxyl functional monomer is made by emulsion polymerization in the presence of the sulfonated polyurethane to produce a latex of hybrid particles. The resulting latex is considered to be one embodiment of the present invention. Such a latex may, if desired, be incorporated into a formulation, as described herein above, having relatively low percent solids and having a continuous liquid medium that contains both water and at least one water-miscible liquid other than water. It is contemplated that in some of such formulations, some or all of the acrylic polymer will be dissolved in the continuous liquid medium. Independently, it is contemplated that in some of such formulations, some or all of the sulfonated polyurethane will be dissolved in the continuous liquid medium.

Independently, in some embodiments involving a continuous liquid medium, the continuous liquid medium contains 19% water or less, by weight based on the weight of the continuous liquid medium. In some of such embodiments, the amount of water, by weight based on the weight of the continuous liquid medium, is 15% or less; or 5% or less; or 1% or less; or 0.1% or less; or none. Such embodiments may be made by any method. One method, for example, would be to first produce a latex of hybrid particles as described herein above. Then, such a latex of hybrid particles could be mixed with a liquid other than water, and some or all of the water in the mixture could be removed. Alternatively, such a latex of hybrid particles could be subjected to drying or other isolation method that removes some or all of the water from the hybrid particles, and then the hybrid particles could be mixed with a liquid other than water.

Among embodiments of the present invention in which acrylic polymer and sulfonated polyurethane are carried in a continuous liquid medium, it is contemplated that, in some embodiments, the composition will be stable over a storage period. That means that, after a storage period, the composition does not show significant amount of separation of solid material, phase separation of liquid phases, other signs of instability, or any combination thereof. In some embodiments, compositions of the present invention are stable over storage periods of 10 minutes or more; or 1 hour or more, or 1 day or more, or 4 days or more; 30 days or more. Independently, in some embodiments, compositions of the present invention are stable over a storage period at 25°C; or 45°C, or 60°C.

Solid material may, in some cases, separate from the composition of the present invention during storage, either by sinking or by floating. The solid material may be collected, dried, and weighted. The solid material may be collected, for example, by methods that are the same as those described herein above for the collection of gel, by filtration, by centrifugation, or by any combination thereof. The weight of separated solid material can be compared to the total weight of solid material in the composition. In some embodiments, the weight of separated solid material, as a percentage of the total weight of solid material in the composition, is 10% or less; or 5% or less; or 1% or less; or 0.5% or less; or none.

In some embodiments in which a composition of the present invention includes a continuous liquid medium, the composition may separate into two or more liquid phases during storage. In some embodiments, no phase separation occurs. If phase separation occurs, the amount of phase separation may be assessed by placing the composition into a vertical cylindrical container and observing the layers formed by the separated phases. Useful characteristics of a phase separated sample are, for example, the total vertical size of the composition and the thickness of the thinnest layer (i.e., the vertical size of the smallest phase-separated liquid phase). In some embodiments, the ratio of the total vertical size of the composition to the vertical size of the smallest phase-separated liquid phase is 5:1 or higher; or 10:1 or higher; or 20:1 or higher; or 50:1 or higher; or 100:1 or higher.

In some embodiments of the present invention, a latex of hybrid particles is made as described herein above, and that latex is stable over a storage period.

In some embodiments, a composition of the present invention is used in a hair styling composition. As used herein, the term "hair styling composition" means a pump or aerosol hair spray, styling gel, styling glaze, spray foam, styling cream, styling wax, styling lotion, liquid foam, spray gel, pomade, blow-dry lotion, curl activator, or mousse that is used on hair to hold the hair in a particular shape or configuration. In some embodiments, the hair styling composition in the present invention is a hair spray. The term "hair" means natural human hair, animal hair, artificial hair, and wigs or hairpieces containing hair.

Among embodiments in which a composition of the present invention is used in an aerosol spray, an appropriate aerosol propellant is also used. Some suitable propellants are, for example, alkanes having 4 or fewer carbon atoms, fluorinated hydrocarbons having 2 carbon atoms, dimethyl ether, other compounds that are gaseous at 25°C at the pressure normally found in an aerosol can, and mixtures thereof. Some suitable propellants are, for example, n-butane,

isobutane, propane, dimethyl ether, 1,1-difluoroethane, tetrafluoroethane, and mixtures thereof.

In some embodiments in which the polymer composition of the present invention is used as part of a hair styling composition that is a liquid at 25°C that is readily pourable or sprayable. The continuous medium of such a hair styling composition is considered herein to be a continuous liquid medium.

Independently, also contemplated are embodiments in which the polymer composition of the present invention is used as part of a hair styling composition that does not readily flow under its own weight at 25°C but is easily spread by hand at 25°C. Some examples of such hair styling compositions are styling gels, some styling creams, and mousses. The continuous medium of such a hair styling composition also is considered herein to be a continuous liquid medium.

Among embodiments in which the polymer composition of the present invention is used as part of a hair styling composition, in some embodiments the hair styling composition has a continuous liquid medium that is a cosmetically acceptable fluid medium. Water or other solvents may be used alone or in mixtures as the continuous liquid medium of a hair styling composition. In some embodiments, the continuous liquid medium is a mixture of water and a water-miscible liquid other than water; such mixtures may have any of the compositions described herein above. For example, some hair styling compositions of the present invention have continuous liquid medium that is 50% to 65% water, by weight based on the weight of the continuous liquid medium. Independently, some hair styling compositions of the present invention have continuous liquid medium that is a mixture of water and ethanol.

Independently, in some embodiments, a polymer composition of the present invention is used as part of a hair styling composition, that hair styling composition has a continuous liquid medium that is a cosmetically acceptable fluid medium, and that fluid medium contains water in an amount, by weight based on the weight of the fluid medium, of 19% or less; or 15% or less; or 5% or less; or 1% or less; or 0.1% or less; or none.

Independently, hair styling compositions may have any of the percent solids values described herein above. For example, some hair styling compositions of the present invention have percent solids of 0.1% or higher; or 0.5% or higher; or 2% or higher; or 4% or higher. Independently, some hair styling compositions of the present invention have percent solids of 10% or lower, or 6% or lower.

Among embodiments in which the polymers of the present invention are used as part of a hair styling composition, the polymer compositions may be present in dispersed form, may be present in dissolved form, or as a combination thereof.

Among embodiments in which the composition of the present invention is used as part of a hair styling composition, the hair styling composition may optionally contain one or more of the following additional ingredients: perfumes, dyestuffs which can color the hair styling composition itself or hair fibers, preservatives, sequestering agents, thickeners, silicones, softeners, foam synergistic agents, foam stabilizers, sun filters, peptizing agents, conditioning agents, shine agents, proteins, herbals, botanicals, neutralizers, plasticizers, and anionic, non-ionic, cationic, or amphoteric surfactants, or mixtures thereof. It is contemplated that any one of or any combination of such additional ingredients may be added to the hair styling composition after the polymer compositions are formed.

In some embodiments, acrylic polymer is formed in the absence of surfactant other than sulfonated polyurethane, and then a hair styling composition is formed that contains that acrylic polymer, that sulfonated polyurethane, and no surfactant other than sulfonated polyurethane. It is contemplated that such hair styling compositions will have advantages (such as, for example, less tendency to corrode metal containers and less tendency to form foam) over hair styling compositions containing common surfactants.

In addition to use in hair styling compositions, the compositions of the present invention are also contemplated for use in other compositions useful in hair care, skin care, cosmetics, or other related uses. For example, the compositions of the present invention are contemplated for use in one or more of hair mask, hair conditioner, hair shampoo, eye mascara, body wash, skin mask, skin lotion, color cosmetics, make-up, lipstick, or other related uses.

In some embodiments, the composition of the present invention contains a small amount of sulfonated polyester polymer or does not contain any sulfonated polyester polymer. As used herein, a polyester polymer is a polymer that has Mw of 5,000 or greater, that has plural ester linking groups in the polymer main chain, and that has no urethane linking groups. An ester linking group is

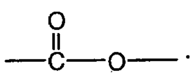

If a polymer has ester linking groups and urethane linking groups, then that polymer is considered herein to be a polyurethane and not a polyester polymer.

As used herein, a "small amount" of polyester polymer means that the ratio of dry weight of polyester polymer to dry weight of sulfonated polyurethane is 0.1:1 or lower; or 0.05:1 or lower; or 0.01:1 or lower.

In some embodiments, the composition of the present invention contains a small amount of sulfonated polyester polymer and contains a small amount of non-sulfonated polyester polymer. In some embodiments, the composition of the present invention contains no sulfonated polyester polymer and contains no non-sulfonated polyester polymer.

Independently, in some embodiments, the composition of the present invention contains no polyester polymer, whether sulfonated or un-sulfonated, that has Mw of 2,000 or greater.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. As a further, independent, example, if a particular parameter is disclosed to have suitable minima of 1, 2, and 3, and if that parameter is disclosed to have suitable maxima of 9 and 10, then all the following ranges are contemplated: 1 to 9, 1 to 10, 2 to 9, 2 to 10, 3 to 9, and 3 to 10.

**EXAMPLES**

Some of the ingredients used in the following examples were as follows:

| Name | Description | Supplier |
|---|---|---|
| SS55-225-130 | sulfonated polyesterdiol containing pendant sodium sulfonate group, Mw of 550 | Crompton Corp., Middlebury, CT |
| Fomrez™ 8066-72 | polyesterdiol (reaction product of isophthalic acid, adipic acid, and hexane diol), Mw of 550 | Crompton Corp., Middlebury, CT |
| BA | butyl acrylate | Rohm and Haas Co. |
| HEMA | hydroxyethyl methacrylate | Rohm and Haas Co. |
| MMA | methyl methacrylate | Rohm and Haas Co. |
| MAA | methacrylic acid | Rohm and Haas Co. |
| n-DDM | n-dodecyl mercaptan | commodity |
| Sancure™ 2104 | carboxylated polyurethane dispersion | Noveon, Cleveland, OH |
| AMP-95 | aminomethyl propanol | Angus Chemical |

Example 1: Synthesis of sulfonated polyurethane dispersion

To a reactor equipped with an overhead stirrer, a reflux condenser, a nitrogen adapter, an addition funnel and a thermocouple was charged 15.75 grams of polyethylene glycol (Mn=1500 g/mol), 43.82 grams of SS55-225-130, 9.59 grams of 2-methyl-1,3-propanediol, and 112 grams of anhydrous methyl ethyl ketone. The mixture was heated to 70 °C, then 46.68 grams of isophorone diisocyanate and eye drop (i.e., one drop from an eyedropper) of dibutyl tin dilaurate were added. The resulting mixture was heated under refluxing conditions for 4 hours. The resulting polyurethane prepolymer was transformed to water-based dispersion through the removal of methyl ethyl ketone and the dispersion in water. The water-based sulfonated polyurethane dispersion had a solids content of 15% and a theoretical sodium sulfonate content of 49 meq/100g of dry polymer.

Example 2: Synthesis of sulfonated polyurethane dispersion

To a reactor equipped with an overhead stirrer, a reflux condenser, a nitrogen adapter, an addition funnel and a thermocouple was charged 31.5 grams of polyethylene glycol (Mn=1500 g/mol), 43.82 grams of SS55-225-130, 8.54 grams of 2-methyl-1,3-propanediol, and 112 grams of anhydrous methyl ethyl ketone. The mixture was heated to 70 °C, then 46.68 grams of isophorone diisocyanate and eye drop of dibutyl tin dilaurate were added. The resulting mixture was heated and dispersed in water as in Example 1. The water-based sulfonated polyurethane dispersion had a solids content of 16% and a theoretical sodium sulfonate content of 44 meq/100g of dry polymer.

Example 3: Synthesis of sulfonated polyurethane dispersion

To a reactor equipped with an overhead stirrer, a reflux condenser, a nitrogen adapter, an addition funnel and a thermocouple was charged 15.75 grams of polyethylene glycol (Mn=1500 g/mol), 87.78 grams of SS55-225-130, 2.28 grams of 2-methyl-1,3-propanediol, and 112 grams of anhydrous methyl ethyl ketone. The mixture was heated to 70 °C,

then 46.68 grams of isophorone diisocyanate and eye drop of dibutyl tin dilaurate were added. The resulting mixture was heated and dispersed in water as in Example 1. The water-based sulfonated polyurethane dispersion had a solids content of 19% and a theoretical sodium sulfonate content of 75 meq/100g of dry polymer.

Comparative Example C4: Synthesis of carboxylated polyurethane dispersion

To a reactor equipped with an overhead stirrer, a reflux condenser, a nitrogen adapter, an addition funnel and a thermocouple was charged 11.72 grams of polyethylene glycol (Mn=1500 g/mol), 120 grams of Fomrez™ 8066-72, 26.64 grams of 2,2-dimethylolpropionic acid (DMPA) and 160 grams of anhydrous methyl ethyl ketone. The mixture was heated to 70 °C, then 66.69 grams of isophorone diisocyanate and eye drop of dibutyl tin dilaurate were added. The resulting mixture was heated and dispersed in water as in Example 1. The water-based carboxylated polyurethane dispersion had a solids content of 31% and a theoretical carboxylate content of 88.3 meq/100g of dry polymer.

Example 5: Synthesis of sulfonated polyurethane/acrylate hybrid dispersion

A reactor was charged with 277.2 grams of sulfonated polyurethane dispersion from Example 1. Separately, a monomer mixture was prepared by mixing 17.4 grams of BA, 32.71 grams of MMA, 6.96 grams of HEMA, 12.53 grams of MAA, and 0.7 gram of n-DDM. An initiator solution was prepared by dissolving 0.28 gram of ammonium persulfate in 7.36 grams of water. With the nitrogen turned on, the reactor and contents at 85 °C, the monomer mixture and the initiator solution were fed over 60 minutes, while maintaining the reactor temperature at 85 °C. After the monomer mixture and initiator feeds were complete, the reaction mixture was "chased" with a ferrous sulfate, t-butyl hydroperoxide, ammonium persulfate, D-isoascorbic acid combination to reduce residual monomer levels. The reaction mixture was then cooled to room temperature and filtered. The sulfonated polyurethane/acrylate hybrid dispersion had a solids content of 28.3%, pH of 3.9, and a particle distribution with a single peak and median at 71 nm.

Example 6: Synthesis of sulfonated polyurethane/acrylate hybrid

Sulfonated polyurethane/acrylate hybrid dispersion was prepared as described in example 5 except polymer dispersion from Example 2 was used. The resulting dispersion had a solids content of 29.1%, pH of 4.1, and a particle distribution with a single peak and with median particle size of 80 nm.

Example 7: Synthesis of sulfonated polyurethane/acrylate hybrid

Sulfonated polyurethane/acrylate hybrid dispersion was prepared as described in example 5 except polymer dispersion of Example 3 was used. The resulting dispersion had a solids content of 31.9%, pH of 4.6, and a particle distribution with a single peak and with median particle size of 71 nm.

Comparative Example C8: Synthesis of carboxylated polyurethane/acrylate hybrid (without carboxyl monomer in acrylic copolymer)

A reactor was charged with 212 grams of Sancure™ 2104 and 100 grams of water. Separately, a monomer mixture was prepared by mixing 9 grams of BA, 14.1 grams of MMA, 6.9 grams of HEMA, and 0.15 gram of n-DDM. An initiator solution was prepared by dissolving 0.12 gram of ammonium persulfate in 50 grams of water. With the nitrogen turned on, the reactor and contents at 85 °C, the monomer mixture and the initiator solution were fed over 60 minutes, while maintaining the reactor temperature at 85 °C. After the monomer mixture and initiator feeds were complete, the reaction mixture was "chased" with a ferrous sulfate, t-butyl hydroperoxide, ammonium persulfate, D-isoascorbic acid combination to reduce residual monomer levels. The reaction mixture was then cooled to room temperature and filtered. The carboxylated polyurethane/acrylate hybrid dispersion had a solids content of 23%, pH of 7.4. The dispersion was stable during polymer preparation but began to phase separate after 1 day.

Comparative Example C9: Synthesis of carboxylated polyurethane/acrylate hybrid

A reactor was charged with 212 grams of Sancure 2104 and 100 grams of water. Separately, a monomer mixture was prepared by mixing 9 grams of BA, 14.1 grams of MMA, 5.4 grams of HEMA, 2 grams of MAA, and 0.15 gram of n-DDM. An initiator solution was prepared by dissolving 0.12 gram of ammonium persulfate in 50 grams of water. With the nitrogen turned on, the reactor and contents at 85 °C, the monomer mixture and the initiator solution were fed over 60 minutes, while maintaining the reactor temperature at 85 °C. In less than 5 minutes after the monomer mixture was fed, gel was formed in the reactor.

Comparative Example C10: Synthesis of carboxylated polyurethane/acrylate hybrid

Attempt was made to prepare carboxylated polyurethane/acrylate hybrid (with methacrylic acid in acrylate copolymer) by following procedure in example 5 with polymer dispersion from Comparative Example C4. In less than 5 minutes after the monomer mixture was fed, gel was formed in the reactor.

Example 11: Properties of Polyurethanes

Polyurethanes were evaluated as follows:

Acid Amount: milliequivalents of acid groups (either carboxylic or sulfonic, depending on the sample) per 100 grams of polymer by dry weight
PS: particle size, median as measured by CHDF, in nanometers
Solution: polyurethane was dissolved in ethanol and water to give a solution of 55 parts by weight ethanol, 40 parts by weight water, and 5 parts by weight polyurethane solids. The appearance of the solution by eye was then recorded
Film: the solution above was pH adjusted with AMP-95 to pH of 7.0 and then allowed to evaporate at room temperature (25°C). The resulting film was evaluated by eye and by hand, as follows:

brittle: film is easily broken when attempting to bend it with the fingers.

flex: it is possible to bend the film without breaking it by exerting significant pressure with the fingers.
v. flex: it is possible to bend the film by exerting only light pressure with the fingers.
no tack: the film feels smooth to the touch
sl. tack: the film feels slightly tacky to the touch.
tack: the film feels significantly tacky to the touch.

Mw: Weight-Average Molecular weight, measured by GPC.
gel: The dispersion was filtered through a 100-mesh screen. The gel was collected and dried and reported as g/quart of polymer dispersion.

| Example No. | acid amount | PS (nm) | solution | film | Mw |
|---|---|---|---|---|---|
| Comparative Ex. C4 | 88.3 | not measured | clear | clear flex no tack | 16,000 |
| Ex. 1 | 49 | 29 | slightly bluish | clear flex no tack | 11,879 |
| Ex 2 | 44 | 29 | clear | clear v flex sl. tack | 22,664 |
| Ex. 3 | 75 | 29 | clear | clear v. flex tack | 9,935 |

Example 12: Properties of Hybrid polymers of Acrylic Polymer and sulfonated polyurethanes

| Example No. | PS (nm) | solution | film | Mw |
|---|---|---|---|---|
| Ex. 5 | 71 | slightly bluish | clear brittle | 42,543 |
| Ex. 6 | 80 | clear | clear sl. flex | 50,361 |
| Ex. 7 | 71 | clear | clear sl. flex | 40,441 |

Further properties of the same hybrid polymers were evaluated as follows:

| Example No. | percent solids | gel |
|---|---|---|
| Ex. 5 | 28.3 | 0.06 |

(continued)

| Example No. | percent solids | gel |
|---|---|---|
| Ex. 6 | 29.1 | 0.09 |
| Ex. 7 | 31.9 | 0.10 |

## Claims

**1.** A polymer composition comprising at least one acrylic polymer and at least one sulfonated polyurethane, wherein said acrylic polymer comprises 5% or more by weight, based on the dry weight of said acrylic polymer, monomer units of at least one carboxyl functional monomer, wherein one or more chain transfer agent is used in making said acrylic polymer.

**2.** A hair styling composition comprising at least one acrylic polymer and at least one sulfonated polyurethane, wherein said acrylic polymer comprises 5% or more by weight, based on the dry weight of said acrylic polymer, monomer units of at least one carboxyl functional monomer.

**3.** A method of making a polymer composition comprising

(A) providing an aqueous dispersion of sulfonated polyurethane;
(B) adding at least one acrylic monomer to said aqueous dispersion of sulfonated polyurethane;
(C) then forming an acrylic polymer by polymerizing said acrylic monomer in the presence of said sulfonated polyurethane, wherein said acrylic polymer comprises 10% or more by weight, based on the dry weight of said acrylic polymer, monomer units of at least one carboxyl functional monomer.

**4.** The method of claim 3, wherein said steps (B) and (C) are conducted in the absence of surfactant other than sulfonated polyurethane.

Europäisches Patentamt
European Patent Office
Office européen des brevets

# EUROPEAN SEARCH REPORT

Application Number
EP 13 17 3342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 5 807 919 A (DUAN YOULU [US] ET AL)<br>15 September 1998 (1998-09-15)<br>* claim 1 *<br>* column 4, lines 53-65 *<br>* column 5, lines 1-5 *<br>* columns 2-3 *<br>* examples 1-3 *<br>----- | 1<br><br>3,4 | INV.<br>C08G18/08<br>C08L75/04<br>C08L51/00<br>A61K8/81<br>A61K8/87<br>C08F265/04<br>C08G18/40<br>C08G18/46<br>C08G18/75<br>A61Q5/06 |
| Y | WO 2005/016999 A (VALSPAR CORP [US]; KILLILEA T HOWARD [US])<br>24 February 2005 (2005-02-24)<br>* claims 1,8 *<br>----- | 3,4 | |
| A | WO 02/09654 A (OREAL [FR])<br>7 February 2002 (2002-02-07)<br>* claims 1,2 *<br>----- | 2 | |
| | | | TECHNICAL FIELDS SEARCHED (IPC) |
| | | | A61K<br>A61Q<br>C08G<br>C08L |

The present search report has been drawn up for all claims

1

| Place of search | Date of completion of the search | Examiner |
|---|---|---|
| The Hague | 18 October 2013 | Pouilley, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number

EP 13 17 3342

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 13 17 3342

The Search Division considers that the present European patent application does not comply with the requirements of unity of invention and relates to several inventions or groups of inventions, namely:

1. claim: 1

A polymer composition comprising at least one acrylic polymer and at least one sulfonated polyurethane, wherein the acrylic polymer comprises at least 5 wt.-% of a carboxyl functional monomer and a chain transfer agent.

---

2. claims: 2-4(partially)

A polymer composition comprising at least one acrylic polymer and at least one sulfonated polyurethane, wherein the acrylic polymer comprises at least 5 wt.-% of a carboxyl functional monomer.

---

## ANNEX TO THE EUROPEAN SEARCH REPORT ON EUROPEAN PATENT APPLICATION NO. EP 13 17 3342

This annex lists the patent family members relating to the patent documents cited in the above-mentioned European search report. The members are as contained in the European Patent Office EDP file on
The European Patent Office is in no way liable for these particulars which are merely given for the purpose of information.

18-10-2013

| Patent document cited in search report | | Publication date | Patent family member(s) | | | Publication date |
|---|---|---|---|---|---|---|
| US 5807919 | A | 15-09-1998 | AU | 4069397 | A | 06-03-1998 |
| | | | US | 5807919 | A | 15-09-1998 |
| | | | US | 6031045 | A | 29-02-2000 |
| WO 2005016999 | A | 24-02-2005 | AU | 2004264525 | A1 | 24-02-2005 |
| | | | AU | 2010202410 | A1 | 01-07-2010 |
| | | | BR | PI0413511 | A | 10-10-2006 |
| | | | CA | 2535417 | A1 | 24-02-2005 |
| | | | CN | 1882637 | A | 20-12-2006 |
| | | | CN | 101880358 | A | 10-11-2010 |
| | | | EP | 1654306 | A1 | 10-05-2006 |
| | | | JP | 2007502350 | A | 08-02-2007 |
| | | | KR | 20060096412 | A | 11-09-2006 |
| | | | US | 2007027249 | A1 | 01-02-2007 |
| | | | US | 2009163651 | A1 | 25-06-2009 |
| | | | WO | 2005016999 | A1 | 24-02-2005 |
| WO 0209654 | A | 07-02-2002 | AU | 8001601 | A | 13-02-2002 |
| | | | EP | 1307176 | A2 | 07-05-2003 |
| | | | JP | 2004517809 | A | 17-06-2004 |
| | | | US | 6517821 | B1 | 11-02-2003 |
| | | | WO | 0209654 | A2 | 07-02-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6093384 A
- US 5698626 A
- US 5753774 A
- US 5629402 A
- US 6517821 B

### Non-patent literature cited in the description

- **FW BILLMEYER, JR.** Textbook of Polymer Science. 1971